# EUROPEAN PATENT SPECIFICATION

(11) **EP 1 419 781 B1**
(45) Date of publication and mention of the grant of the patent: **23.06.2010**
(21) Application number: 03025973.3
(22) Date of filing: 13.11.2003
(51) Int. Cl.: A61K 36/21, A61K 36/708, A61P 1/02

(54) **Odontostomatological use of plant compounds based on rhubarb roots (species belonging to the Rheum genus) and spinach leaves (spinacia oleracea l.)**
Odontostomatologische Verwendung von Pflanzen-Komponenten auf Basis von Rhabarberwurzel (rheum genera) und Spinatblätter (spinacia oleracea l.)
Usage odontostomatologique des composés d'origine vegetale à base de racines de rhubarbe (genre rheum) et feuilles d'épinard (spinacia oleracea l.)

(30) Priority: 14.11.2002 IT mi20022408
(43) Date of publication of application: 19.05.2004
(73) Proprietor: ABOCA S.p.A., 52037 Sansepolcro (Arezzo) (IT)
(72) Inventor: Mongiorgi, Romano, 40043 Marzabotto (BO) (IT)
(74) Representative: Minoja, Fabrizio

(56) References cited:
- DATABASE CA [Online] CHEMICAL ABSTRACTS SERVICE, COLUMBUS, OHIO, US; GORTNER, ROSS A., JR. ET AL: "Some effects of dietary oxalate on the teeth of white rats" retrieved from STN Database accession no. 40:33871 HCA XP002267573 & JOURNAL OF NUTRITION (1946), 32, 121-31 ,
- DATABASE CA [Online] CHEMICAL ABSTRACTS SERVICE, COLUMBUS, OHIO, US; MCCAY, C. M. ET AL: "Erosion of molar teeth by acid beverages" retrieved from STN Database accession no. 44:8192 HCA XP002267574 & JOURNAL OF NUTRITION (1949), 39, 313-24 ,
- DATABASE WPI Section Ch, Week 197921 Derwent Publications Ltd., London, GB; Class A96, AN 1979-40060B XP002267575 & SU 615 931 A (MOSC SVOBODA COSMETIC), 19 June 1978 (1978-06-19)
- DATABASE WPI Section Ch, Week 199740 Derwent Publications Ltd., London, GB; Class B04, AN 1997-426023 XP002267576 & CN 1 115 248 A (AI R), 24 January 1996 (1996-01-24)
- HIDAKA S ET AL: "Effects of a rhubarb (Rhei rhizoma) solution and its fractions on the formation of calcium phosphate precipitates." JOURNAL OF PERIODONTAL RESEARCH. DENMARK AUG 1996, vol. 31, no. 6, August 1996 (1996-08), pages 408-413, XP009024109 ISSN: 0022-3484

## Description

This invention relates to the use of non-toxic water-soluble plant extracts for the preparation of odontostomatological medicaments. In particular, the invention relates to the application of a series of plant compounds with specific properties in the fields of dentistry and dental hygiene. These products are particularly effective in the treatment of dentinal hypersensitivity.

Dentinal hypersensitivity is a symptom characterised by "pain originating from the dentine in response to thermal, mechanical (tactile), chemical and osmotic stimuli which are not explained by dental disease or lesions" [Addy M, Dental Clinics of North America, 34, 503-514, 1990]. This clinical condition is caused by exposure of the dentine to the oral environment. The numerous epidemiological studies of dentinal sensitivity demonstrate that this clinical condition affects over 15% of the population aged between 20 and 40 years old, with a particular incidence among women.

The sites most often affected by dentinal hypersensitivity are the cervico-buccal areas, especially in the canines and premolars [Absi E. G. et al., J. Clin. Periodontol., 14, 280-284, 1987; Dowel P., et al. J. Clin. Periodontol., 10, 341-350, 1983; Addy M, Dental Clinics of North America, 34, 503-514, 1990].

Any dental treatment involving removal of enamel and dentine (and therefore opening of the dentinal tubules) also inevitably leads to increased sensitivity. Consequently, a patient who presents dentinal hypersensitivity also has an area of exposed dentine (devoid of enamel and/or gingival cover) with the tubular orifices open and no type of surface debris or "smear layer" (the layer of debris that normally covers the dentine and occludes the tubules with "smear plugs") which (wholly or partly) obstructs the dentinal tubules [Gwinnet A.J., Oper. Dent., 3 (Suppl.), 475-481, 1984; Pashley D.H., Oper. Dent., (Suppl.) 13-24, 1984].

An important role in causing the onset or recurrence of dentinal sensitivity is played by the diet. Numerous foods and drinks have acid properties due to their citric, phosphoric or maleic acid content. The presence of these acids can easily cause chemical removal of the surface smear layer, thus opening the dentinal tubules.

Approx. 80% of patients who undergo resective periodontal surgery complain of dentinal sensitivity, which also occurs during scaling procedures.

As the presence of open dentinal tubules is the pre-requisite for the onset of dentinal hypersensitivity, the most accredited treatment basically aims to occlude those tubules. A different therapeutic approach, which aims to reduce receptor sensitivity, involves increasing the intratubular concentration of potassium and strontium ions (by means of topical application) in order to make the dentinal nerve fibres less excitable.

According to the state of the art, the preferable treatment is out-patient treatment, performed directly on the tooth or teeth involved, again with the main objective of occluding the open dentinal tubules. Various techniques and materials have been proposed, depending on the severity of the symptoms. They fall into four main groups: (1) - Mechanical treatment of the sensitive surface to promote the formation of a new smear layer; (2) - Application of chemical agents able to occlude the dentinal tubules either directly or by formation of insoluble precipitates; (3) - Impregnation and consequent obstruction of the dentinal tubules with adhesives; (4) - Application of restoration materials, possibly with the application of slow-fluoride-release intraoral devices. Nowadays, dentists prefer not to place products with available fluorine in contact with the dentine, because fluorine seems to destabilise the dentine lattice slightly, and consequently to act as an undesirable contributory factor in the formation of tooth decay [Mongiorgi R. et al., Odontostomatologia, 24, 6, 528-566, 1998].

As regards the first treatment specified above, cleaning of the dentine surface with silicone or rubber cups, with the application of an abrasive paste, causes the formation of a thin, even smear layer that closes the tubules. This simple treatment does not last, however, because the smear layer is soluble in an acid environment and is therefore easily be removed by patients themselves as a result of incorrect brushing and/or acid drinks. The treatment is therefore used in mild cases, with no history of dentinal sensitivity.

The second type of out-patient treatment is performed by applying chemical substances, specifically oxalates (of potassium, iron, aluminium, ammonium or oxalic acid) [Mongiorgi R. et al., Boll. Soc. It. Biol. 67, 403-407, 1991; Mongiorgi R. et al., Atti 4° Convegno R.S.A.A.F., Bologna, 4, 1991; Mongiorgi R. et al., XXIII Congr. Naz. S.I.O.C.M.F., II/283-II288, 1992; Prati C. et al.., Odontostomatologia, 6, 507509, 1992], which interact with the calcium in the dental apatite to form insoluble microcrystals of calcium oxalate [Mongiorgi R. et al.., Boll. Soc. It. Sper., 68, 99-103, 1992; Mongiorgi R. et al., Archs oral Biol. 39, suppl. 152S, 1994] on the surface of and inside the dentinal tubules.

However, problems may arise due to the toxicity of oxalates.

This technique includes the direct application of ultramicronised apatite (more or less crystalline calcium phosphates) as described, for example, in Italian patent no. 1,271,874, wherein 70% of powdered product granules are under 1 µm (0.2-1 µm).

US 4,634,589 and EP 0346957 describe the use of crystalline apatite in particles of under 8 µm with a concentration of 15% in weight in the first case, and dimensions of between 1 and 15 µm, associated with potassium and/or strontium (as a further destabilising agent), in the second. If these latter materials are capable of bonding, they can only create an occlusive layer on the dentinal wall, without penetrating into the tubules.

The most effective out-patient treatment impregnates the dentine with adhesive resins (this is known as the "resin impregnation technique"). This technique [Nordenvall K.J. et al., J. Prosthet. Dent., 44, 630-637, 1980] became highly effective with the advent of modern dentinal adhesive systems constituted by highly hydrophilic resins able to penetrate a few tens of microns into the tubules. According to the state of the art, the technique of impregnating the exposed dentinal surface with resin is most suitable for patients in whom only a few teeth present average or serious hypersensitivity.

As regards home treatment, there are numerous toothpastes and gels on the market specifically designed for sensitive teeth, with active constituents such as strontium chloride hexahydrate, potassium nitrate, potassium citrate and stannous fluoride, and periodontal gels and mouthwashes have also been proposed [Prati C. et al., Recent Advances in Dental Res. in Italy, Baiesi Ed. 1, 111-117, 1998; Mongiorgi R. et al., Recent Advances in Dental Res. in Italy, Baiesi Ed., 1, 125-132, 1998; Prati C. et al., Recent Advances in Dental Res. in Italy, Baiesi Ed., 1, 133-141]. The data reported in the literature demonstrate that many toothpastes also have a certain desensitising action, often unrelated to the type of active constituent they contain [Gillam D. G. et al., J. Periodontol., 67, 737-742, 1996]. Very often, the other constituents of the toothpaste also play an important part, because they are able to occlude the dentinal tubules. Some experts also believe that the placebo effect plays a crucial part.

In addition to the established out-patient treatments reported above and the home treatments which have become traditional, the use of calcium phosphates in amorphous form (in addition to the micronised and ultramicronised apatite materials already mentioned) has been proposed in order to remineralise the dental tissues and reduce dentinal sensitivity. US 5,268,167 describes the use of amorphous calcium phosphates (ACP), amorphous calcium phosphate fluorides (ACPF) and amorphous calcium carbonate phosphates (ACCP) for this purpose. Similarly, WO 94/04460 proposes amorphous calcium carbonate phosphate fluoride (ACCPF) as a new compound of particular interest for this purpose. These materials are perfectly compatible, both biologically and structurally, with the dental tissues, and seem able to integrate effectively and stably with the dentine. However, they have the drawback of being very hygroscopic, which makes it difficult to include them in gels or toothpastes.

Non-toxic plant compounds have now been discovered which are soluble in an aqueous environment, compatible with the dental tissues and able to combat dentinal sensitivity in an effective, lasting way by occluding and cementing the tubules in depth, and performing a desensitising action that resists attack by chemically aggressive agents present in the oral cavity such as acid foods and drinks.

The plant compounds according to the invention are obtainable from species of rhubarb *(Rheum genus)*, in particular from rhubarb roots, and from *Spinacia oleracea L*., in particular the leaves thereof, either alone or in association thereof.

This invention consequently relates to pharmaceutical compositions containing the said plant compounds and to their use to make medicinal products for the treatment of dentinal hypersensitivity.

The term "plant compound" means any phytotherapeutic product obtainable from plant material by means of one or more transformation processes such as grinding, extraction, concentration, infusion, freeze-drying, centrifugation, filtration or, in general, any process that concentrates and/or purifies and/or extracts the active ingredients from the plant and/or makes it suitable for administration in a galenical, pharmaceutical or cosmetic form.

These techniques are well-known and widely used in the medicinal plant sector.

The rhubarb and spinach compounds are preferably obtained by extraction of the roots and leaves respectively with water or alcoholic or hydroalcoholic solutions. Soft or dry extracts can be used as well as liquid extracts.

According to a preferred method of preparation of the plant compounds, rhubarb root and spinach leaves in the fresh or dry state (humidity under 12%) are selected and chopped to a particle size suitable to undergo the extraction process.

The suitably chopped plant is then introduced into perforated racks and loaded into extractors, namely stainless steel containers in which the solvent circulates. The plant/solvent ratio varies according to the initial state of the plant (fresh or dried) and ranges between 1:11 and 1:15 in the case of a dried plant and between 1:3 and 1:6 in the case of a fresh plant.

The extraction takes place at temperature of between 40 and 60°C, and typically lasts for 6 hours.

The extract obtained is filtered, and then reduced in volume under vacuum until a concentrate with a dry residue of between 10 and 30% w/w is obtained.

The concentrated extract is dried, preferably by freeze-drying.

During the freezing stage the temperature of the product is reduced to -40 to -45°C very rapidly to promote fine crystallisation. The formation of tiny crystals prevents the creation of demixing in the fluid and favourably affects the final structure of the lyophilisate and its rehydration at the time of use.

The freeze-dried extract contains a concentrate of the active constituents of the original plant in an unpurified form, ie. with other components soluble in the extractive solution.

The freeze-dried extracts thus obtained have the following content of characterising elements:

### (Freeze-dried rhubarb root extract)

Potassium content: minimum 2%
Total acid content calculated as malic acid: minimum 4%.

### (Freeze-dried spinach leaf extract):

Potassium content: minimum 15%
Total acid content calculated as malic acid: minimum 1%.

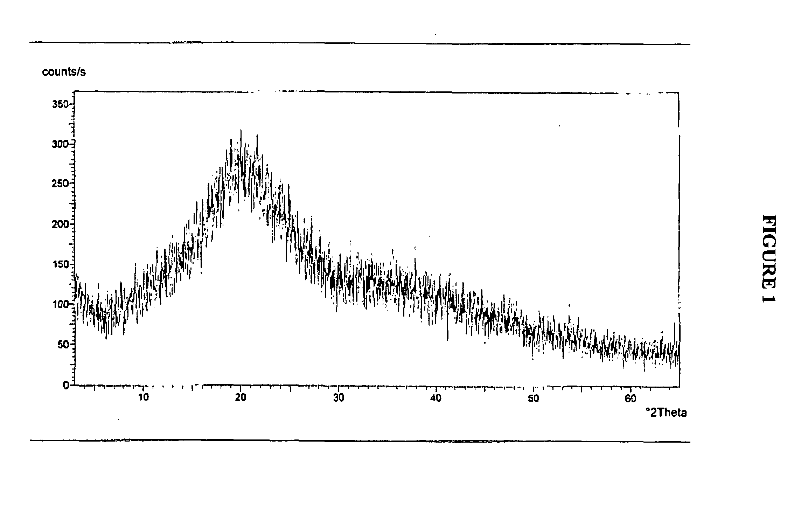

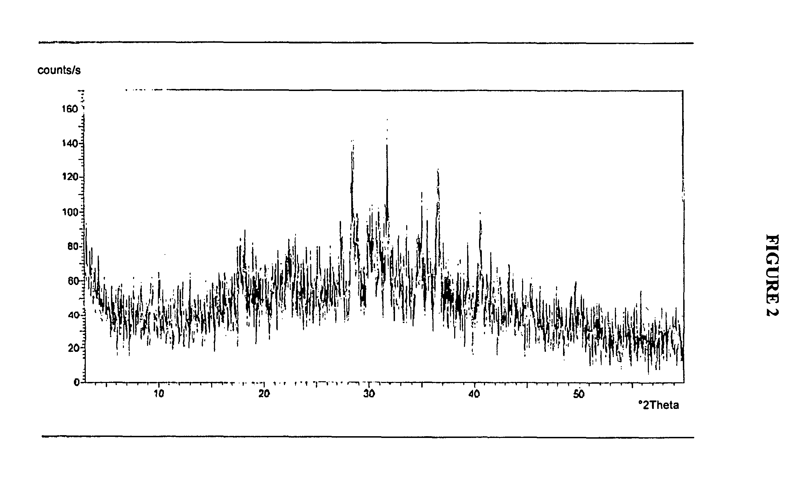

The structural characteristics of the plant compounds described above (whether crystalline or not) are demonstrated by the diffractograms shown in Figures 1 and 2.

Freeze-drying is advantageous because it eliminates the large protein molecules present which could otherwise increase sensitivity to osmotic phenomena, as demonstrated by Pashley et al. [Archs. Oral Biol. Vol. 29, no. 9, pp. 725-728, 1984].

It is also possible to obtain a fine powder with the required active constituent concentration which is structurally amorphous or partly crystalline, soluble, and compatible with the dental tissues.

In view of the characteristics of the product as a whole, which react with calcium apatite to give insoluble calcium salts, it is easy to obtain partial mechanical filling of the tubules (which are easily reached) due to the salts that form and crystallise *in situ*, and a microcrystalline layer generalised all over the dentinal surface, with consequent elimination or drastic reduction of dentinal hypersensitivity.

The compositions for odontostomatological use of the invention contain the plant compounds described above, possibly with additional ingredients and excipients of the type normally used in preparations for the oral cavity (such as sodium fluoride), or other plant compounds used to flavour the formulation and/or enrich it with potassium, provided that they do not contain calcium ions. The compositions, which are preferably in the form of a paste, gel, mouthwash, spray solution, sweets, chewing gum, cream or powder, for local use, preferably contain 0.5 to 50% in weight of rhubarb or spinach compounds, or more preferably a mixture of the two. The compositions of the invention can be advantageously used to protect the dentine, treat dentinal hypersensitivity, close the dentinal tubules or reduce their functional diameter and protect prosthesis posts, and can also be used as bases and linings for endodontal filling cements, orthodontic cements and sealants for enamel and dentine.

The invention is illustrated in greater detail in the examples below.

### EXAMPLE 1

### Toothpaste:

Composition: Al(OH)₃ [35 g], CH₃(CH₂)₁₁OSO₃ [2 g], Carboxymethylcellulose sodium salt [2 g], rhubarb compounds [15 g], glycerin [13 g], sorbitol [13 g], water deionised with parabens [q.s. for 100 g].

### EXAMPLE 2

### Gel:

The gel obtained derives from combining PEG 400 and PEG 4000 hot, in the proportion of 55:45. 100 grams of this gel contain 20 grams of Spinach Compound.

### EXAMPLE 3

### Solution:

An aqueous solution was obtained (with deionised water and parabens) containing 25% of a 1:1 mixture of spinach and rhubarb compounds.

### EXAMPLE 4

### Dentinal permeability tests

The efficacy of the plant compounds according to the invention in treating dentinal hypersensitivity was evaluated in terms of reduction of the hydraulic conductance in the dentinal tubules, following a well-established protocol [*Pashley, 1990, loc. cit*.] in accordance with the international literature. Healthy human molars, extracted from young patients for orthodontic reasons, were used to conduct this test. Each tooth was suitably separated from the root and dissected to obtain a segment of crown devoid of occlusal enamel; the pulp tissue was removed and the segment was secured with adhesive to a Plexiglas support, with the flat occlusal dentine surface facing upwards. A tubular steel segment ran through the entire thickness of the support and emerged in the pulp chamber to effect the hydraulic connection, below the Plexiglas support level, with the hydraulic conductance detection system. The said detection system consisted of a simple hydrodynamic device constituted by a set of capillary chambers filled with deionised water and connected through the tubular steel segment to the pulp chamber. The passage of water from the pulp chamber to the occlusal surface through the dentine was demonstrated by the movement of an air bubble in a graduated microcapillary tube located in the hydrodynamic system. The tests reported below were conducted with the device described after thorough washing of the occlusal dentine surface, to which the active constituents tested were added from time to time. The various active constituents tested were added to the paste and gel according to the composition described in the preceding examples, as follows:
1 - blank 1, constituted by the paste vehicle only;
2 - blank 2, constituted by the gel vehicle only;
3 - paste + 20% rhubarb extract;
4 - paste + 20% spinach extract;
5 - gel + 20% rhubarb extract;
6 - gel + 20% spinach extract;
7 - 25% solution in weight of rhubarb extract without vehicle;
8 - 25% solution in weight of spinach extract without vehicle;
9 - paste + rhubarb extract (20%) + spinach extract (20%);
10 - gel + rhubarb extract (20%) + spinach extract (20%).

Each test comprised the following steps:
- Formation of a smear layer by rubbing on abrasive paper under manual pressure, followed by washing with deionised water;
- Application of 0.5 M EDTA (ethylenediamenetetraacetic acid, Sigma, St. Louis, USA) at pH 7.4 for 5 minutes. Permeability was measured after leaving the sample to stand for 2 minutes and washing it. The value obtained was taken as reference in the subsequent tests, associated with 100% permeability.
- Treatment with the blanks and with the solution, paste e gel containing the plant compounds in the various compositions, by painting the tooth for 3 minutes; this time was chosen on the basis of the correct duration of brushing during normal teeth cleaning operations. The tooth was painted rather than brushed with a toothbrush to prevent the formation of a smear layer caused by brushing, which would have made it difficult to evaluate the efficacy of the product.
- Etching with 37% orthophosphoric acid in weight (Merck, Darmstadt) for 1½ minutes, followed by rinsing. Permeability was evaluated after one minute.

The permeability tests were conducted 7 times (each test comprising 4 measurements) for each of the times established after treatment with each preparation, and each of the steps listed above was repeated.

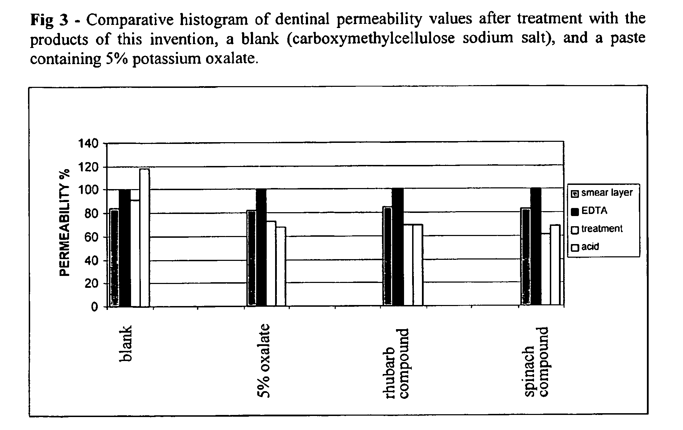

The results obtained (final average) are set out in the Table for all steps of the test, while the behaviour of the basic plant compounds compared with the blank, the reference compound and acid attack (data relating to the measurement taken 3 min. after application of the product and 1.5 min. after acid treatment with phosphoric acid) is shown in the histogram in Fig. 3.

### See (Fig. 3)

**T A B L E - experimental dentinal permeability values. Each value is a percentage compared with EDTA, which expresses 100% permeability. The pastes and gels contain 20% of the plant compound, while the solutions contain 25%. The basic paste has the same composition as that used for the experimental comparison containing Merck 5% potassium oxalate.**

| | After formation of smear layer | Treatment with EDTA | After application of product for 3 min. | After acid treatment for 1.5 min. |
|---|---|---|---|---|
| Paste blank | 84.3 | 100 | 91.6 | 118.4 |
| Gel blank | 85.4 | 100 | 98.8 | 127.2 |
| Merck 5% K oxalate reference paste | 82.7 | 100 | 72.7 | 68.3 |
| Rhubarb compound | 85.3 | 100 | 69.7 | 69.5 |
| Spinach compound | 83.6 | 100 | 61.6 | 68.9 |

The following conclusions can be drawn from the above Table, and are also evident from the histogram: there are no favourable findings with the blanks (paste and gel in the formulations stated in the examples set out above, on which commercial toothpastes are based), and the dentine remains highly permeable and particularly liable to acid attack. Permeability values exceeding 100% are observed after treatment with H₃PO₄ (see histogram).

Dentinal permeability is considerably reduced from 100% permeability after treatment with EDTA to 72.7% with the use of Merck potassium oxalate (5%), and further improved to 68.3% after acid attack. Even acid attack does not produce any appreciable adverse effect in this case due to the action of potassium oxalate which, by chelating the calcium of the dentine, produces a calcium oxalate precipitate insoluble in acid that partly or wholly occludes the dentinal tubules. Despite these favourable effects, the efficacy of this possible treatment is cancelled out by the toxicity of the synthetic product.

Rhubarb and spinach extracts reduce dentinal permeability considerably, in a similar way to the reference product. Moreover, acid does not affect the efficacy of this treatment because the dicarboxylic acids present in these plants form insoluble salts on contact with dentine. This demonstrates that these plant extracts can be usefully presented as biocompatible materials (because of their widespread use in the food industry) which are very active in reducing hydraulic conductance in the dentinal tubules for a long time, and stably integrate with dentinal tissues in depth.

## Claims

1. Pharmaceutical compositions containing as active ingredients an association of liquid, solid or dried extracts of rhubarb *(Rheum genus)* and *Spinacia oleracea L.,* mixed with suitable excipients.

2. Pharmaceutical compositions as claimed in claim 1, wherein the extracts are obtained by hydroalcoholic extraction from rhubarb roots and *Spinacia oleracea L.* leaves.

3. Pharmaceutical compositions as claimed in claims 1 or 2 in the form of paste, gel, mouthwash, spray solution, sweets, chewing gum, solution or powder.

4. Use of an association of liquid, solid or dried extracts of rhubarb *(Rheum genus)* and *Spinacia oleracea L.* compounds for the preparation of medicaments for the treatment of dentinal hypersensitivity.

## Patentansprüche

1. Eine pharmazeutische Zusammensetzung enthaltend als Wirkstoff eine Ansammlung aus flüssigen, festen oder getrockneten Extrakten von Rhabarber *(Rheum genus)* und *Spinacia oleracea L.,* gemischt mit geeigneten Hilfsstoffen.

2. Pharmazeutische Zusammensetzung gemäß Anspruch 1, worin die Extrakte durch eine alkoholisch-wässrige Extraktion der Rhabarberwurzeln und der Blätter von *Spinacia oleracea L.* gewonnen werden.

3. Pharmazeutische Zusammensetzung gemäß Anspruch 1 oder 2 in Form einer Paste, eines Gels, eines Mundwassers, einer Sprühlösung, von Süßigkeiten, von Kaugummi, einer Lösung oder eines Pulver.

4. Verwendung einer Ansammlung von flüssigen, festen oder getrockneten Extrakten von Rhabarber *(Rheum genus)* und *Spinacia oleracea L.* Komponenten für die Herstellung von Medikamenten zur Behandlung von Dentin-Überempfindlichkeit.

## Revendications

1. Compositions pharmaceutiques contenant en tant qu'ingrédients actifs une association d'extraits liquides, solides ou secs, de rhubarbe (*Rheum genus*) et de *Spinacia oleracea L.,* mélangés avec des excipients appropriés.

2. Compositions pharmaceutiques selon la revendication 1, dans lesquelles les extraits sont obtenus par extraction hydroalcoolique à partir de racines de rhubarbe et de feuilles de *Spinacia oleracea L..*

3. Compositions pharmaceutiques selon la revendication 1 ou 2 sous la forme d'une pâte, d'un gel, d'un dentifrice, d'une solution en spray, de bonbons, d'un chewing-gum, d'une solution ou d'une poudre.

4. Utilisation d'une association d'extraits liquides, solides ou secs, de composés de rhubarbe (*Rheum genus)* et de *Spinacia oleracea L.* pour la préparation de médicaments pour le traitement d'hypersensibilité dentaire.
